(19) Europäisches Patentamt
European Patent Office
Office européen des brevets

(11) **EP 2 833 129 B1**

(12) **EUROPEAN PATENT SPECIFICATION**

(45) Date of publication and mention
of the grant of the patent:
**09.10.2019 Bulletin 2019/41**

(51) Int Cl.:
**G01N 27/12** *(2006.01)*        **G01N 33/00** *(2006.01)*

(21) Application number: **13003799.7**

(22) Date of filing: **30.07.2013**

(54) **Method and apparatus for analyzing a gas by a conductance-type particulate metal-oxide gas sensor**

Verfahren und Vorrichtung zur Analyse von Gasen durch einen auf Leitfähigkeit basierenden Partikulat-Metalloxid-Gassensor

Procédé et dispositif pour l'analyse de gaz avec un capteur de conductivité à base de particules-oxyde de métal

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.02.2015 Bulletin 2015/06**

(73) Proprietor: **Sensirion AG**
**8712 Stäfa (CH)**

(72) Inventor: **Bühler, Johannes**
**8610 Uster (CH)**

(74) Representative: **Mirza, Akram Karim**
**E. Blum & Co. AG**
**Vorderberg 11**
**8044 Zürich (CH)**

(56) References cited:
**US-A1- 2009 312 954**

• KHATKO V ET AL: "Gas sensors based on nanoparticle WO3 thick films", PROCEEDINGS OF IEEE SENSORS 2004 : [IEEE SENSORS 2004 CONFERENCE] ; OCTOBER 24 - 27, VIENNA UNIVERSITY OF TECHNOLOGY, VIENNA, AUSTRIA, IEEE SERVICE CENTER, PISCATAWAY, NJ, 24 October 2004 (2004-10-24), pages 188-191, XP010793366, ISBN: 978-0-7803-8692-1

• MULLER G ET AL: "A MEMS toolkit for metal-oxide-based gas sensing systems", THIN SOLID FILMS, ELSEVIER-SEQUOIA S.A. LAUSANNE, CH, vol. 436, no. 1, 22 July 2003 (2003-07-22) , pages 34-45, XP004431389, ISSN: 0040-6090, DOI: 10.1016/S0040-6090(03)00523-6

• GOPEL W ET AL: "SnO2 sensors: current status and future prospects", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 26, no. 1-3, 1 January 1995 (1995-01-01), pages 1-12, XP027261074, ISSN: 0925-4005 [retrieved on 1995-01-01]

• N. BARSAN; D. KOZIEJ; U. WEIMAR: "Metal oxide-based gas sensor research: How to?", SENSORS AND ACTUATORS B, vol. 121, 2007, pages 18-35, XP5856163,

• LANGE U ET AL: "Separated analysis of bulk and contact resistance of conducting polymers: Comparison of simultaneous two- and four-point measurements with impedance measurements", JOURNAL OF ELECTROANALYTICAL CHEMISTRY AND INTERFACIAL ELECTROCHEMISTRY, ELSEVIER, AMSTERDAM, NL, vol. 622, no. 2, 15 October 2008 (2008-10-15), pages 246-251, XP025471379, ISSN: 0022-0728, DOI: 10.1016/J.JELECHEM.2008.06.013 [retrieved on 2008-06-17]

## Description

Technical Field

The invention relates to a method for analyzing a gas. It also relates to a gas sensing device for carrying out this method.

Background Art

A method and sensor for detecting an analyte in a gas is described in GB 2464016. It uses a patch of metal oxide that changes its electrical conductivity depending on the composition of the gas that it is exposed to. The patch is heated to a suitable operating temperature, typically in the range of 300°C - 600°C. The patch is arranged on a membrane for thermal insulation and thermally coupled to a heater.

The sensor is adapted to detect the presence, in particular the concentration, of an analyte by processing the current flowing through the electrodes.

Metal oxides are, however, sensitive to a number of different gaseous analytes, which makes it difficult to identify the analyte that a gas sensor is measuring.

Khatko V. et al, "Gas Sensors Based on Nanoparticle WO3 Thick Films", Proceedings of IEEE Sensors 2004; pp. 188 - 191 (ISBN 978-0-7803-8692-1) describes measurements on chemical sensors based on nanoparticle $WO_3$ thick films doped with indium and bismuth. The sensors are operated at different temperatures in order to classify different analytes.

US 2009/0312954 describes a gas sensor using a CVD metal-oxide layer. Sensors with different electrode gaps are integrated in a single substrate in order to distinguish sheet resistance and contact resistance.

Disclosure of the Invention

The problem to be solved by the present invention is therefore to provide a method and sensing device that allows to distinguish between several analytes in a gas and/or to measure analyte concentrations more accurately. In this context, the term "distinguishing between several analytes in a gas" is to be understood as a step of identifying a single analyte species (or group of species) from a plurality of possible species (or groups of species) in a gas, or to detect which species (or groups of species) are present in the gas. It may also, but not necessarily, include the step of determining concentrations or amounts of the detected species (or groups of species).

This problem is solved by the method and sensing device of the independent claims.

Accordingly, the method for analyzing a gas comprises the following steps:

- A particulate metal-oxide sensing material forming a chemiresistor is exposed to the gas to be analysed. In this context, the term "particulate metal-oxide sensing material" designates a sensing material formed by at least 90% of metal-oxide particles. Electrodes are applied to the sensing material.
- A plurality of measurements is carried out by applying different voltages over said electrodes. Each measurement yields a measured value indicative of a conductance of said sensing material. At least some of the measurements differ in the strength of the electrical field generated by the electrodes in the sensing material.
- The measured values obtained in said measurements are analyzed for distinguishing between several analytes present in said gas and/or for measuring a concentration of at least one analyte.

This method is based on the understanding that the conductance of a particulate metal oxide sensing material depends on the strength of the applied electrical field, i.e. the current-versus-voltage characteristic of such a sensing material is typically non-linear, and this non-linearity depends on the species of analytes that are present. Hence, by combining the conductance values measured for different electrical fields, it becomes possible to distinguish between the various analytes and/or to measure a concentration more accurately.

Advantageously, the sensing material comprises an aggregate of metal-oxide particles, wherein said aggregate of metal-oxide particles is arranged between at least two of said electrodes.

Especially advantageous materials to be used in the metal-oxide particles are metal-oxide semiconductors, which can include tin oxide, tungsten oxide, gallium oxide, indium oxide, or zinc oxide or mixtures thereof in either not-intentionally doped or doped form, and in particular comprising at least one of the materials selected from the group of tin oxide (in particular $SnO_2$) and tungsten oxide (in particular $WO_3$).

To carry out measurements with differing electrical fields in the sensing material, the applied voltage is varied. In addition thereto, at least part of said plurality of measurements can be carried out on a plurality of different sensing units. Each sensing unit comprises two electrodes arranged at a gap distance, with the sensing material arranged between said electrodes. At least two of the sensing units have differing gap distances so that, even with equal applied voltages, the electrical fields in the different sensing units differ. In particular the gap distances between a largest and a smallest gap distance of said sensing units differ by at least 50% of a largest one of said gap distances in order to obtain clearly different electrical fields.

The invention also relates to a gas sensing device for carrying out the method above. Such a gas sensing device comprises:

- A gas sensor comprising electrodes and a particulate metal-oxide sensing material in contact with the electrodes. The metal-oxide sensing material forms a chemiresistor.
- A detection unit comprising a voltage or current source connected to said electrodes and an analyzer for determining measured values indicative of the conductance of the sensing material.

Further, the sensing device comprises a control unit that is adapted and structured to
carrying out a plurality of measurements by applying different voltages over said electrodes, wherein each measurement yields one of said measured values, wherein said measurements differ in the strength of an electrical field applied to said sensing material, and
combining said measured values for distinguishing several analytes present in said gas and/or for measuring a concentration of at least one analyte in the gas.

Other advantageous embodiments are listed in the dependent claims as well as in the description below.

Brief Description of the Drawings

The invention will be better understood and objects other than those set forth above will become apparent from the following detailed description thereof. Such description makes reference to the annexed drawings, wherein:

Fig. 1 is a perspective view of a portable electronic gas sensing device,
Fig. 2 is a top view of a gas sensor,
Fig. 3 is a block circuit diagram of the gas sensing device,
Fig. 4 shows current and voltage traces for a tin-oxide sensor in air of 50% relative humidity with 10 ppm carbon monoxide, and
Fig. 5 shows current and voltage traces for a tin-oxide sensor in air of 50% relative humidity with 10 ppm ethanol.

Modes for Carrying Out the Invention

*Definitions:*

A "chemiresistor" is a device that changes its electrical conductance as a function of the composition of the gas surrounding it.

The "average" particle diameter of the sensing material is defined as the mathematical average or median diameter of the particles that are forming the sensing material.

*Device design:*

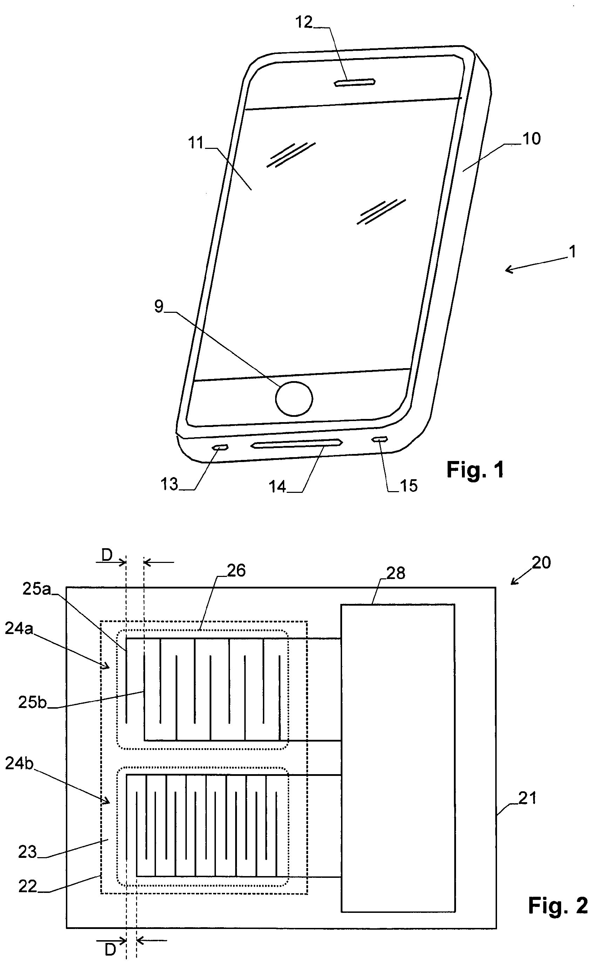

The gas sensing device of Fig. 1 is a portable electronic device 1, such as a mobile phone, a portable computer or a tablet computer. The housing 10 of the mobile phone includes a front side with a display 11 and input elements like buttons 9 to let a user interact with the phone. Also shown on the front side is an opening 12 for a loudspeaker. Further openings 13, 14 are located at a lower side wall of the housing 10. It is well known to mount components like microphones and loudspeakers behind such openings. A further opening 15, which is e.g. also arranged on the lower side wall of housing 10, provides access to a gas sensor as described in more detail below.

Fig. 2 shows a top view of the gas sensor 20. It comprises a substrate 21, which is advantageously a silicon substrate. A recess or opening 22 is arranged on the substrate, with a membrane 23 spanning it. Membrane 23 defines a region of low thermal conductance for locating the actual sensing units 24a, 24b of gas sensor 20. For a single sensing unit, this device design is e.g. known form GB 2464016.

Each sensing unit 24a, 24b comprises a pair of electrodes 25a, 25b, which can e.g. be interdigitated electrodes as shown. The electrodes, which are advantageously gold or platinum electrodes, are covered by and in electrical contact with a patch of sensing material 26. The sensing material is a particulate metal-oxide and consists of an aggregate of metal-oxide particles arranged on top of and between the electrodes 25a, 25b. The sensing material is a chemiresistor material that changes its electrical resistance depending on the composition of the gas surrounding it.

Advantageous sensing materials are doped or undoped metal-oxide semiconductors, such as tin oxide and tungsten oxide, albeit other suitable metal oxide materials are known to the skilled person.

As can be seen, the two sensing units have different gap distances D between the electrodes 25a, 25b. Typical gap distances are in the order of 1 to 100 $\mu$m, in particular 2 and 20 $\mu$m.

Further, gas sensor 20 advantageously comprises a circuitry 28 integrated e.g. in CMOS technology on substrate 21. Such circuitry can e.g. comprise analog components for amplifying sensing signals or for driving the heaters of the gas sensor and digital components for digitally processing the signals and controlling the operation of the gas sensor.

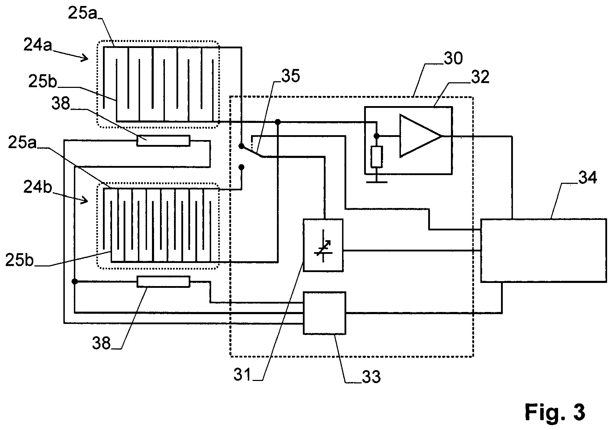

Fig. 3 shows a block diagram showing the (in the present context) most relevant parts of gas sensing device 1.

As can be seen, the device comprises a detection unit 30, which includes a voltage or current source 31, an analyzer 32 and a heater control 33. Further, the device comprises a control unit 34. Typically, detection unit 30 forms part of circuitry 28 integrated on substrate 20. Control unit 34 may e.g. be implemented in part in the circuitry 28 on substrate 20 and in part in hard- and/or software in the processing circuits of gas sensing device 1, such as in a microprocessor running in sensing device 1.

Voltage or current source 31 is advantageously a variable voltage or current source, wherein the current or voltage to be generated is controlled by control unit 34. In the embodiment of Fig. 3, a variable voltage source is used. It is connected, e.g. by means of a switch 35, to a first electrode 25a of either one of the sensing units 24a, 24b. Alternatively, separate current or voltage sources 31 can be provided for each sensing unit 24a, 24b. In yet another embodiment, voltage or current source 31 can be applied to the first electrode 25a of all sensing units 24a, 24b at the same time without a selective switch 35 between the source and the first electrodes (i.e. the first electrodes 25a are electrically connected to each other) if at least some of the sensing units 24a, 24b have different electrode gap distances D. In this case, the individual sensing units 24a, 24b can be measured by arranging an analogue demultiplexer between the second electrodes 25b of the sensing units 24a, 24b and analyzer 32, or several analyzers can be provided with each analyzer being connected to one sensing unit 24a, 24b.

In the shown embodiment, analyzer 32 is e.g. connected to the second electrode 25b of all the sensing units 24a, 24b. In the embodiment of Fig. 3, where a voltage source 31 is used, analyzer is adapted to measure the current flowing between the electrodes 25a, 25b of each sensing unit 24a, 24b.

Heater control 33 is connected to heaters 38 that are adapted to heat the sensing material 26 at each sensing unit 24a, 24b to an operating temperature. For metal oxide sensing units, typical operating temperatures are in the range of 150°C - 500°C. The temperature generated by temperature control 33 can be set by control unit 34. The heaters 38 may e.g. be implemented as electrical leads in a metal layer of membrane 23.

*Operation:*

As mentioned above, sensing device 1 is adapted to carry out a plurality of measurements, wherein at least some of these measurements differ in the strength of the electrical field generated by the electrodes 25a, 25b in the sensing material 26.

This is based on the understanding that the current-voltage characteristics of particulate metal-oxide gas sensors are non-linear and the non-linearity depends on the composition of the gas that the sensing material is in contact with.

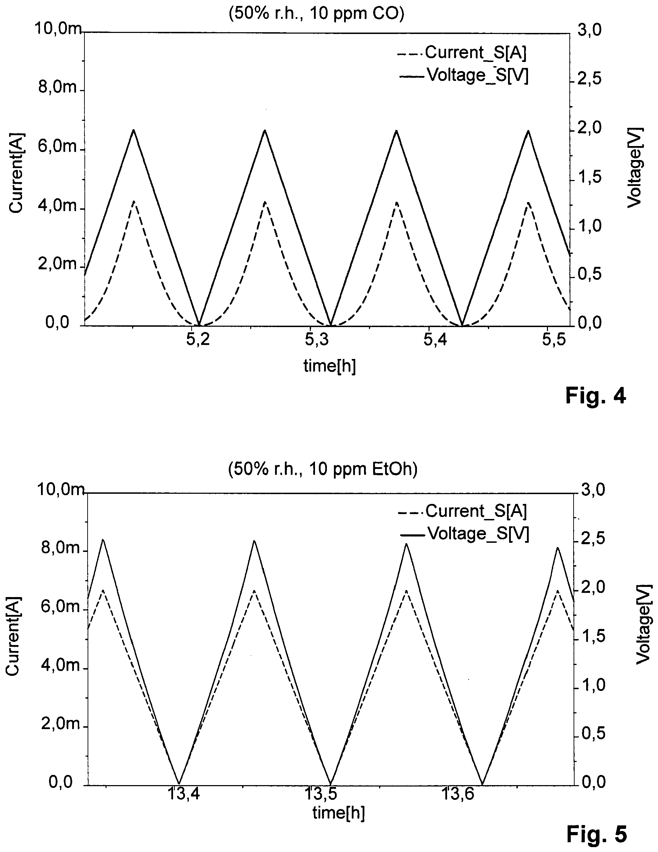

This is illustrated in Figs. 4 and 5, which show the response of a tin oxide sensor to carbon monoxide and ethanol, respectively, at 300°C. In this experiment, the voltage was varied between, approximately, 0 and 2 Volts in a sawtooth curve of a period of approximately 7 minutes, and the current through the sensor was measured. As can be seen, an atmosphere containing 10 ppm CO evokes a strongly non-linear current response, while the current response in an atmosphere with only ethanol is substantially linear.

In other words, if the value $p_u$ measured by analyzer 32 is indicative of (i.e. a function of) the conductance (i.e. the ratio between current and voltage) of a sensing unit u, the gas sensing device carries out N measurements with

$$p_{u,n} = F_u\,(E_{u,n},\ c_1,\ \ldots\ c_M) \hspace{2cm} (1$$

where $u = 1 \ldots U$ is the index of the sensing unit, $n = 1 \ldots N$ is the index of the experiment, $p_{u,n}$ is the value measured for experiment n, $E_{u,n}$ is the electrical field applied to sensing unit u in experiment n (where $E_{u,n}$ can be approximated by $U_{u,n}/D_u$ with $U_{u,n}$ being the voltage applied in experiment n and $D_u$ being the electrode gap separation of sensing unit u). $c_1, \ldots c_M$ are the concentrations of the analytes (or groups of analytes) of interest in the gas surrounding the sensing material. F is a vector-valued function describing the dependence of $P_{u,n}$ on $E_{u,n}$, $c_1, \ldots c_M$. Function F can e.g. be found heuristically by taking a series of calibration measurements for differing, known concentrations $c_1, \ldots c_M$ and known values of $E_{u,n}$ and by measuring the resulting values p. Methods for designing function F and finding its parameters, e.g. by using a polynomial for F and by fitting the polynomial coefficients to the results of the calibration measurements, are known to the skilled person.

Note that a given experiment does not necessarily have to carry out a measurement on all sensing units.

Hence, control unit 34 is adapted and structured to carry out a sufficiently large number N of measurements for differing fields E, thereby generating a set of equations (1). Then, control unit 34 can determine the concentrations $c_1, ... c_M$, e.g. by minimizing the sum of the squares of the errors in the equations (1) by varying the values $c_1, ... c_M$ in order to derive a set of best matching values $c_1, ... c_M$.

As described by N. Barsan, D. Koziej, and U. Weimar in "Metal oxide-based gas sensor research: How to?" in Sensors and Actuators B 121 (2007), pp. 18 - 35, the non-linearities of the current-voltage characteristics of particulate metal-oxide sensing layers are, to a large degree, formed by the need of the charge carriers to cross the particle boundaries. According to this publication, a particulate metal-oxide sensing layer can be modelled as a series of potential barriers, with the barriers being formed by the particle boundaries. Typically, most of the potential applied by the electrodes will drop over the particle boundaries because the bulks of the particles typically have much higher conductance than the boundaries. According to Barsan et al., non linearities are observed when the potential drop per barrier is comparable to k·T, with k being the Boltzmann constant and T the operating temperature of the sensing layer. Hence, in a first approximation, strong non-linearities will typically set in when

$$e \cdot U \cdot d/D > k \cdot T/20. \hspace{3cm} (2)$$

wherein

    k is the Boltzmann constant,
    e is the electron charge,
    T is a temperature of said sensing material,
    U is a voltage between said electrodes,
    d is an average diameter of said particles and
    D is a gap distance between said electrodes.

Eq. (2) divides k·T by a factor of 20 because it has been found that non-linearities will already be present even when the potential difference over the barriers is still somewhat below k·T/e and also because U·d/D is only an approximation of the voltage over each barrier.

It must be noted that even though the above publication by Barsan et al. recognizes the non-linearity of the current-voltage characteristics, i.e. the voltage-dependence of the conductance, this non-linearity is identified as a problem to be avoided, but not as a means for distinguishing different species of gases.

The value of the potential difference k·T/e/20 at 300°C is 0.0025 V. Typical voltages available in modern low-power integrated circuits are in the order of 1 V. Hence, in order to obtain a voltage drop of 0.0025 V per boundary, there must be around 400 grain boundaries between the electrodes. Hence, the corresponding ratio D/d between the electrode gap D and the average particle diameter d should be around 400. If D/d is much larger than that, the non-linearities will be observed at higher voltages only. If D/d is much smaller, the number of grain boundaries will be so small that there would be substantial manufacturing differences from one device to another. Hence, an advantageous range of D/d is between 50 and 1000.

*Notes:*

In the above embodiment, gas sensor 20 comprises two sensing units 24a, 24b having different electrode gap widths D in order to generate different electrical fields in the sensing material, and they are driven by a variable current or voltage source 31. when a variable current or voltage source 31 is used, gap width D can be the same for all sensing units, or a single sensing unit can be used, because it is possible to vary the electrical field within the sensing material by changing the current or voltage.

Using two or more sensing units 24a, 24b is advantageous because it allows to carry out the measurements more quickly.

In summary, the techniques described above allow to distinguish several analytes in a mixture of gases by carrying out conductance measurements on a particulate metal-oxide chemiresistor sensing layer at different voltages (or electrical fields). Since the current-voltage characteristics of such sensors are non-linear and the non-linearitiy depends on the gaseous analytes, this type of measurement allows to distinguish between a plurality of analytes.

While there are shown and described presently preferred embodiments of the invention, it is to be distinctly understood that the invention is not limited thereto but may be otherwise variously embodied and practised within the scope of the following claims.

**Claims**

1. A method for analyzing a gas comprising the step of exposing a particulate metal-oxide sensing material (26), which forms a chemiresistor, to said gas, wherein electrodes (25a, 25b) are applied to said sensing material (26), and wherein said method is further **characterized by** the steps of
carrying out a plurality of measurements by applying different voltages over said electrodes (25a, 25b), wherein each measurement yields a measured value indicative of a conductance of said sensing material (26), wherein at least some of said measurements differ in the strength of an electrical field generated by said electrodes (25a, 25b) in said sensing material (26), and
combining the measured values obtained in said experiments for distinguishing between several analytes and/or measure a concentration of at least one analyte present in said gas.

2. The method of claim 1 wherein said sensing material (26) comprises an aggregate of metal-oxide particles, and wherein said aggregate of metal-oxide particles is arranged between at least two of said electrodes (25a, 25b).

3. The method of claim 2 wherein said plurality of measurements comprises at least one measurement where

$$e \cdot U \cdot d/D \; > \; k \cdot T/20$$

wherein

k is the Boltzmann constant,
e is the electron charge,
T is a temperature of said sensing material (26),
U is the voltage applied between said electrodes (25a, 25b),
d is an average diameter of said particles and
D is a gap distance between said electrodes (25a, 25b).

4. The method claim 3 wherein D/d is between 50 and 1000, wherein d is an average diameter of said particles and D is a gap distance between said electrodes (25a, 25b).

5. The method of any of the claims 3 or 4, wherein said metal-oxide particles are of a semiconducting metal oxide.

6. The method of any of the preceding claims wherein said metal-oxide particles comprise a material selected a group comprising tin oxide, tungsten oxide, gallium oxide, indium oxide, or zinc oxide or mixtures thereof in either not-intentionally doped or doped form.

7. The method of any of the preceding claims wherein at least some of said plurality of measurements are carried out on a plurality of sensing units (24a, 24b), wherein each sensing unit (24a, 24b) comprises two electrodes (25a, 25b) arranged at a gap distance, wherein said sensing material (26) is arranged between said electrodes (25a, 25b) and wherein at least two sensing units (24a, 24b) having differing gap distances are used.

8. The method of claim 7 wherein a largest and a smallest gap distance of said gap distances differ by at least 50% of a largest one of said gap distances.

9. The method of any of the preceding claims wherein said metal-oxide sensing material (26) is in contact with said electrodes (25a, 25b).

10. A gas sensing device for carrying out the method of any of the preceding claims comprising
a gas sensor (20) comprising electrodes (25a, 25b) and a particulate metal-oxide sensing material (26) forming a chemiresistor in contact with said electrodes (25a, 25b),
a detection unit (30) comprising a voltage or current source (31) connected to said electrodes (25a, 25b) and an analyzer (32) for determining measured values indicative of a conductance of said sensing material (26), and
a control unit (34) **characterized by** being adapted to
carrying out a plurality of measurements by applying different voltages over said electrodes (25a, 25b), wherein each measurement yields one of said measured values, wherein said measurements differ in the strength of an electrical field applied to said sensing material, and

combining said measured values for distinguishing several analytes present in said gas and/or for measuring a concentration of at least one analyte.

11. The device of claim 10 wherein said sensing material (26) comprises an aggregate of metal-oxide particles having an average diameter d, wherein said aggregate of metal-oxide particles is arranged between two electrodes (25a, 25b) and wherein D/d is between 50 and 1000, wherein d is an average diameter of said particles and D is a gap distance between said electrodes (25a, 25b) .

12. The device of any of the claims 10 or 11, wherein said metal-oxide sensing material (26) comprises tin oxide, tungsten oxide, gallium oxide, indium oxide, or zinc oxide or mixtures thereof in either not-intentionally doped or doped form.

13. The device of any of the claims 10 to 12 further comprising a semiconductor substrate (21), wherein said gas sensor at least part of said detection unit (30) and/or said control unit (34) are integrated on said semiconductor substrate.

14. The gas sensing device of claim 10, wherein said gas sensing device is selected from a group comprising:

    a mobile phone,
    a handheld computer,
    an electronic reader,
    a tablet computer,
    a game controller,
    a pointing device,
    a photo or a video camera,
    a digital music player,
    a wrist watch,
    a key fob,
    a head set, and
    a computer peripheral.

**Patentansprüche**

1. Ein Verfahren zum Analysieren eines Gases, umfassend den Schritt des Aussetzens eines teilchenförmigen Metalloxid-Sensormaterials (26), das einen Chemiresistor bildet, an das Gas, wobei Elektroden (25a, 25b) auf das Sensormaterial (26) aufgebracht werden, und wobei das Verfahren ferner durch die folgenden Schritte gekennzeichnet ist
Durchführen einer Vielzahl von Messungen durch Anlegen unterschiedlicher Spannungen an die Elektroden (25a, 25b), wobei jede Messung einen Messwert ergibt, der eine Leitfähigkeit des Sensormaterials (26) anzeigt, wobei sich mindestens einige der Messungen in der Stärke eines von den Elektroden (25a, 25b) erzeugten elektrischen Feldes in dem Sensormaterial (26) unterscheiden, und
Kombinieren der in den Experimenten erhaltenen Messwerte zum Unterscheiden zwischen mehreren Analyten und/oder Messen einer Konzentration von mindestens einem in dem Gas vorhandenen Analyten.

2. Das Verfahren nach Anspruch 1, wobei das Sensormaterial (26) ein Aggregat aus Metalloxidteilchen umfasst, und wobei das Aggregat aus Metalloxidteilchen zwischen mindestens zwei der Elektroden (25a, 25b) angeordnet ist.

3. Das Verfahren nach Anspruch 2, wobei die Vielzahl von Messungen mindestens eine Messung umfasst, mit

$$e \cdot U \cdot d/D > k \cdot T/20$$

wobei

    k die Boltzmann-Konstante ist,
    e die Elektronenladung ist,
    T eine Temperatur des Sensormaterials (26) ist,
    U die Spannung ist, die zwischen den Elektroden (25a, 25b) angelegt wird,
    d ein durchschnittlicher Durchmesser der Partikel ist und

D ein Spaltabstand zwischen den Elektroden (25a, 25b) ist.

4. Das Verfahren nach Anspruch 3, wobei D/d zwischen 50 und 1000 liegt, wobei d ein durchschnittlicher Durchmesser der Partikel ist und D ein Spaltabstand zwischen den Elektroden (25a, 25b) ist.

5. Das Verfahren nach einem der Ansprüche 3 oder 4, wobei die Metalloxidteilchen aus einem halbleitenden Metalloxid bestehen.

6. Das Verfahren nach einem der vorhergehenden Ansprüche, wobei die Metalloxidteilchen ein Material umfassen, das ausgewählt ist aus einer Gruppe, die Zinnoxid, Wolframoxid, Galliumoxid, Indiumoxid oder Zinkoxid oder Mischungen davon in entweder nicht beabsichtigt dotierter oder dotierter Form umfasst.

7. Das Verfahren nach einem der vorhergehenden Ansprüche, wobei mindestens einige der mehreren Messungen auf einer Vielzahl von Sensoreinheiten (24a, 24b) durchgeführt werden, wobei jede Sensoreinheit (24a, 24b) zwei Elektroden (25a, 25b) umfasst, die in einem Spaltabstand angeordnet sind, wobei das Sensormaterial (26) zwischen den Elektroden (25a, 25b) angeordnet ist und wobei mindestens zwei Sensoreinheiten (24a, 24b) mit unterschiedlichen Spaltabständen verwendet werden.

8. Das Verfahren nach Anspruch 7, wobei sich ein grösster und ein kleinster Spaltabstand der Spaltabstände um mindestens 50% von einem grössten der Spaltabstände unterscheiden.

9. Das Verfahren nach einem der vorhergehenden Ansprüche, wobei das Metalloxid-Sensormaterial (26) in Kontakt mit den Elektroden (25a, 25b) steht.

10. Eine Gassensorvorrichtung zur Durchführung des Verfahrens nach einem der vorhergehenden Ansprüche, umfassend

einen Gassensor (20), der Elektroden (25a, 25b) und ein teilchenförmiges Metalloxid-Sensormaterial (26) umfasst, das einen Chemiresistor in Kontakt mit den Elektroden (25a, 25b) bildet,

eine Erfassungseinheit (30), die eine mit den Elektroden (25a, 25b) verbundene Spannungs- oder Stromquelle (31) und einen Analysator (32) zum Bestimmen von Messwerten umfasst, die eine Leitfähigkeit des Sensormaterials (26) anzeigen, und

eine Steuereinheit (34), **dadurch gekennzeichnet, dass** sie ausgebildet ist zum

Durchführen einer Vielzahl von Messungen durch Anlegen unterschiedlicher Spannungen an die Elektroden (25a, 25b), wobei jede Messung einen der Messwerte ergibt, wobei sich die Messungen in der Stärke eines an das Sensormaterial angelegten elektrischen Feldes unterscheiden, und

Kombinieren der Messwerte zum Unterscheiden mehrerer im Gas vorhandener Analyten und/oder zum Messen einer Konzentration von mindestens einem Analyten.

11. Die Vorrichtung nach Anspruch 10, wobei das Sensormaterial (26) ein Aggregat aus Metalloxidteilchen mit einem durchschnittlichen Durchmesser d umfasst, wobei das Aggregat aus Metalloxidteilchen zwischen zwei Elektroden (25a, 25b) angeordnet ist und wobei D/d zwischen 50 und 1000 liegt, wobei d ein durchschnittlicher Durchmesser der Teilchen ist und D ein Spaltabstand zwischen den Elektroden (25a, 25b) ist.

12. Die Vorrichtung nach einem der Ansprüche 10 oder 11, wobei das Metalloxid-Sensormaterial (26) Zinnoxid, Wolframoxid, Galliumoxid, Indiumoxid oder Zinkoxid oder Mischungen davon in entweder nicht beabsichtigt dotierter oder dotierter Form umfasst.

13. Die Vorrichtung nach einem der Ansprüche 10 bis 12, weiter umfassend ein Halbleitersubstrat (21), wobei der Gassensor, mindestens ein Teil der Detektionseinheit (30) und/oder die Steuereinheit (34) auf dem Halbleitersubstrat integriert sind.

14. Die Gassensorvorrichtung nach Anspruch 10, wobei die Gassensorvorrichtung ausgewählt ist aus einer Gruppe umfassend:

ein Handy,
einen Handheld-Computer,
ein elektronisches Lesegerät,
einen Tablet-Computer,

einen Spielecontroller,
eine Zeigevorrichtung,
eine Foto oder Videokamera,
einen digitalen Musik-Player,
eine Armbanduhr,
einen Schlüsselanhänger,
ein Headset und
ein Computerperipheriegerät.

**Revendications**

1. Un procédé d'analyse d'un gaz comprenant l'étape d'exposition d'un matériau de détection d'oxyde métallique particulaire (26), qui forme une chimiorésistance, audit gaz, dans lequel des électrodes (25a, 25b) sont appliquées audit matériau de détection (26), et dans lequel ledit procédé est davantage **caractérisé par** les étapes suivantes Effectuer une pluralité de mesures en appliquant différentes tensions sur lesdites électrodes (25a, 25b), dans lequel chaque mesure donne une valeur mesurée indicative d'une conductance dudit matériau de détection (26), dans lequel au moins certaines desdites mesures diffèrent par l'intensité d'un champ électrique généré par lesdites électrodes (25a, 25b) dans ledit matériau de détection (26), et Combiner les valeurs mesurées obtenues dans lesdites expériences pour distinguer entre plusieurs analytes et/ou mesurer une concentration d'au moins un analyte présent dans ledit gaz.

2. Le procédé selon la revendication 1, dans lequel ledit matériau de détection (26) comprend un agrégat de particules d'oxyde métallique, et dans lequel ledit agrégat de particules d'oxyde métallique est disposé entre au moins deux desdites électrodes (25a, 25b).

3. Le procédé selon la revendication 2, dans lequel ladite pluralité de mesures comprend au moins une mesure où

$$e \cdot U \cdot d/D > k \cdot T/20$$

dans laquelle

k est la constante de Boltzmann,
e est la charge électronique,
T est une température dudit matériau de détection (26),
U est la tension appliquée entre lesdites électrodes (25a, 25b),
d est un diamètre moyen desdites particules et
D est une distance entre lesdites électrodes (25a, 25b).

4. Le procédé selon la revendication 3, où D/d est compris entre 50 et 1000, où d est un diamètre moyen desdites particules et D est une distance entre lesdites électrodes (25a, 25b).

5. Le procédé selon l'une quelconque des revendications 3 ou 4, dans lequel lesdites particules d'oxyde métallique sont constituées d'un oxyde métallique semi-conducteur.

6. Le procédé selon l'une quelconque des revendications précédentes, dans lequel lesdites particules d'oxyde métallique comprennent un matériau choisi parmi un groupe comprenant l'oxyde d'étain, l'oxyde de tungstène, l'oxyde de gallium, l'oxyde d'indium ou l'oxyde de zinc ou leurs mélanges sous forme non intentionnellement dopée ou dopée.

7. Le procédé selon l'une quelconque des revendications précédentes, dans lequel au moins une partie de ladite pluralité de mesures est effectuée sur une pluralité d'unités de détection (24a, 24b), dans lequel chaque unité de détection (24a, 24b) comprend deux électrodes (25a, 25b) disposées à une distance de fente, dans lequel ledit matériau détecteur (26) est disposé entre lesdites électrodes (25a, 25b) et dans lequel au moins deux unités de détection (24a, 24b) présentant des distances différentes sont utilisées.

8. Le procédé selon la revendication 7, dans lequel une plus grande et une plus petite distance de fente desdites distances de fente diffèrent d'au moins 50% d'une plus grande desdites distances de fente.

9. Le procédé selon l'une quelconque des revendications précédentes, dans lequel ledit matériau de détection d'oxyde métallique (26) est en contact avec lesdites électrodes (25a, 25b).

10. Un dispositif de détection de gaz pour mettre en oeuvre le procédé selon l'une quelconque des revendications précédentes comprenant
un capteur de gaz (20) comprenant des électrodes (25a, 25b) et un matériau de détection d'oxyde métallique particulaire (26) formant une chimiorésistance en contact avec lesdites électrodes (25a, 25b),
une unité de détection (30) comprenant une source de tension ou de courant (31) connectée auxdites électrodes (25a, 25b) et un analyseur (32) pour déterminer des valeurs mesurées indiquant une conductance dudit matériau de détection (26), et
une unité de commande (34) **caractérisée en ce qu'**elle est adaptée pour
L'exécution d'une pluralité de mesures en appliquant différentes tensions sur lesdites électrodes (25a, 25b), dans laquelle chaque mesure donne l'une desdites valeurs mesurées, dans laquelle lesdites mesures diffèrent par l'intensité d'un champ électrique appliqué audit matériau de détection, et
La combinaison desdites valeurs mesurées pour distinguer plusieurs analytes présents dans ledit gaz et/ou pour mesurer une concentration d'au moins un analyte.

11. Le dispositif selon la revendication 10, dans lequel ledit matériau de détection (26) comprend un agrégat de particules d'oxyde métallique ayant un diamètre moyen d, dans lequel ledit agrégat de particules d'oxyde métallique est disposé entre deux électrodes (25a, 25b) et dans lequel D/d est entre 50 et 1000, d étant un diamètre moyen desdites particules et D étant une distance de fente entre lesdites électrodes (25a, 25b).

12. Le dispositif selon l'une quelconque des revendications 10 ou 11, dans lequel ledit matériau de détection d'oxyde métallique (26) comprend de l'oxyde d'étain, de l'oxyde de tungstène, de l'oxyde de gallium, de l'oxyde d'indium ou de l'oxyde de zinc ou leurs mélanges sous forme non intentionnellement dopée ou dopée.

13. Le dispositif selon l'une quelconque des revendications 10 à 12, comprenant en outre un substrat semi-conducteur (21), dans lequel ledit capteur de gaz, au moins une partie de ladite unité de détection (30) et/ou ladite unité de commande (34) sont intégrées sur ledit substrat semi-conducteur.

14. Le dispositif de détection de gaz selon la revendication 10, dans lequel ledit dispositif de détection de gaz est choisi dans un groupe comprenant:

   un téléphone portable,
   un ordinateur de poche,
   un lecteur électronique,
   un ordinateur tablette,
   un contrôleur de jeu,
   un dispositif de pointage,
   une photo ou une caméra vidéo,
   un lecteur de musique numérique,
   une montre-bracelet,
   un porte-clés,
   un jeu de tête, et
   un périphérique informatique.

**Fig. 1**

**Fig. 2**

**Fig. 3**

(50% r.h., 10 ppm CO)

Fig. 4

(50% r.h., 10 ppm EtOh)

Fig. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2464016 A

- US 20090312954 A

**Non-patent literature cited in the description**

- **KHATKO V. et al.** Gas Sensors Based on Nanoparticle WO3 Thick Films. *Proceedings of IEEE Sensors,* 2004, ISBN 978-0-7803-8692-1, 188-191

- **N. BARSAN ; D. KOZIEJ ; U. WEIMAR.** Metal oxide-based gas sensor research: How to?. *Sensors and Actuators B,* 2007, vol. 121, 18-35